# EUROPEAN PATENT APPLICATION

(11) **EP 4 335 841 A1**
(43) Date of publication of application: **13.03.2024**
(21) Application number: 21942432.2
(22) Date of filing: 28.05.2021
(51) Int. Cl.: C07D 291/06, B01J 27/053

(54) **PREPARATION METHOD FOR ACESULFAME POTASSIUM**

(71) Applicant: Anhui Jinhe Industrial Co., Ltd., Chuzhou, Anhui 239200 (CN)
(72) Inventor: ZHOU, Rui, Chuzhou, Anhui 239200 (CN); DING, Zhen, Chuzhou, Anhui 239200 (CN); CHEN, Yongxu, Chuzhou, Anhui 239200 (CN); YANG, Fengbao, Chuzhou, Anhui 239200 (CN); LIU, Gang, Chuzhou, Anhui 239200 (CN)
(74) Representative: Ipside
(86) International application number: PCT/CN2021/097009
(87) International publication number: WO 2022/246861

(57) **Abstract**

Described herein is a method for preparing acesulfame potassium, comprising: adding triethylamine to a sulfamic acid solution, and carrying out an amination reaction to produce a sulfamic acid ammonium salt solution; adding diketene to the obtained sulfamic acid ammonium salt solution, and under the action of a solid superacid catalyst, carrying out an acylation reaction to obtain an intermediate solution; dissolving sulfur trioxide in a solvent to form a cyclizing agent solution; adding the cyclizing agent solution to the intermediate solution, and carrying out a sulfonation cyclization reaction to obtain a cyclized product solution; adding a hydrolysis agent to the cyclized product solution, and carrying out a hydrolysis reaction to obtain a hydrolysis product solution; and adding a potassium hydroxide solution to the organic phase of the hydrolysis product solution to obtain acesulfame potassium.

## Description

### Technical Field

The present disclosure belongs to the technical field of fine chemical manufacturing, and specifically relates to a method for preparing acesulfame potassium.

### Background of the Invention

Acesulfame potassium (acesulfame), also known as AK sugar, is a widely used sugar substitute food additive and has white crystalline powder appearance. It is a kind of organic synthetic salt, with a taste similar to sugarcane, easily soluble in water, slightly soluble in alcohol, stable in chemical properties, and not prone to decomposition failure. It does not participate in body metabolism, and does not provide energy; it has high sweetness, and the price is low; it does not cause dental caries; and it is stable to heat and acid.

At present, in the synthesis of acesulfame, a diketene-sulfur trioxide method is generally used, and the specific reacting steps thereof comprise: reacting sulfamic acid with an amine to form an amino sulfamate, and then reacting the amino sulfamate with a diketene to form an acetyl acetamide salt; subjecting the acetyl acetamide salt to a cyclization reaction in the presence of sulfur trioxide to form a cyclic sulfur trioxide adduct; hydrolyzing the cyclic compound to obtain a hydrolysate (ASH); and then treating the hydrolysate with potassium hydroxide to obtain acesulfame potassium (ASK).

At present, in the synthesis of acesulfame, a diketene-sulfur trioxide method is generally used, and the specific reaction steps thereof comprise: reacting sulfamic acid with an amine to form an amino sulfonate, and then reacting the amino sulfamate with a diketene to form an acetyl acetamide salt; subjecting the acetyl acetamide salt to a cyclization reaction in the presence of sulfur trioxide to form a cyclic sulfur trioxide adduct; hydrolyzing the cyclic compound to obtain a hydrolysate (ASH); and then treating the hydrolysate with potassium hydroxide to obtain acesulfame potassium (ASK).

On one hand, acetic acid needs to be added as a catalyst in the reaction process, which makes acetic acid impurities remain in the final acesulfame product, resulting in poor color of acesulfame and affecting the use experience of people.

On the other hand, in the hydrolysis step of the cyclic compound hydrolysis is generally carried out under acidic conditions at present, which has the defects of low yield, instability and easy decomposition of the cyclized product. Although some techniques have been studied for this, there remains a problem of low yield and poor final product quality.

For example, the Chinese patent document CN107868064A discloses a method for synthesizing an acesulfame precursor cyclic compound (ASH) in a microchannel reactor, the application is mainly about carrying out cyclization and hydrolysis reactions of a reaction intermediate acylating agent and a cyclizing agent at certain concentrations by means of a microchannel reactor, and the total yield of the product thereof is only 45-60%. The disadvantage of this process is that sulfur trioxide is unstable under reaction conditions, the speed of cyclization reaction is not controlled, and side reactions are easy to occur.

As another example, the Chinese patent document CN108191790A discloses sulfonated microchannel reaction method and device for acesulfame production, processing intermediate raw materials and cyclizing agent raw material in first and second cyclization microreactors respectively, and then exchanging heat by a heat exchanger. Since the reaction time is long, the cyclized product is easy to decompose, the yield of the hydrolysis reaction is low, the yield of the obtained finished product of acesulfame potassium is low, and the quality of the finished product is poor.

### Summary of the Invention

In view of the above-mentioned problems, the present application provides a method for preparing acesulfame potassium which overcomes the above-mentioned problems or at least partially solves the above-mentioned problems.

According to an aspect of the present application, a method for preparing acesulfame potassium is provided, which comprises:
an intermediate preparation step: adding triethylamine to a sulfamic acid solution, and carrying out an amination reaction to produce an ammonium sulfamate solution; adding diketene to the obtained ammonium sulfamate solution, and carrying out an acylation reaction under the action of a solid superacid catalyst to obtain an intermediate solution;
a sulfonation cyclization step: dissolving sulfur trioxide in a solvent to form a cyclizing agent solution; adding the cyclizing agent solution to the intermediate solution, and carrying out a sulfonation cyclization reaction to obtain a cyclized product solution;
a hydrolysis step: adding a hydrolysis agent to the cyclized product solution, and carrying out a hydrolysis reaction to obtain a hydrolysate solution; and
a salt forming step: adding potassium hydroxide to the organic phase of the hydrolysate solution to obtain acesulfame potassium.

Optionally, in the above method, the solid superacid catalyst is a SO₄²⁻/MₓO_{y} type catalyst, wherein M represents a metal atom.

Optionally, in the above method, the solid superacid catalyst is an SO₄²⁻/Fe₂O₃ type catalyst.

Optionally, in the above method, the hydrolysis agent is deionized water or an ethanol aqueous solution.

Optionally, in the above method, the hydrolysis agent is the ethanol aqueous solution, wherein the mass concentration of ethanol in the ethanol aqueous solution is 30-65%.

Optionally, in the above method, the reaction temperature of the hydrolysis reaction is 0 to 20° C, preferably 10 to 20° C; and the reaction time of the hydrolysis reaction is 30-400 s.

Optionally, in the above method, the ratio of the amount of sulfamic acid to the amount of sulfur trioxide is 4-8: 1, preferably 6: 1; and the solvent is dichloromethane.

Optionally, in the above method, the ratio of the amount of sulfur trioxide to the amount of water in the hydrolysis agent is 1: 1-4, preferably 1: 1-1.5.

Optionally, in the above method, the specific steps of the intermediate preparation step are as follows:
dissolving sulfamic acid in a first solvent to prepare a first reaction solution;
dissolving triethylamine in a second solvent to prepare a second reaction solution, and adding the second reaction solution into the first reaction solution for an amination reaction to form an ammonium sulfamate solution;
dissolving diketene in a third solvent a third reaction solution; and
filling the reactor with a solid superacid catalyst, sequentially adding the ammonium sulfamate solution and the third reaction solution to the reactor, and reacting under a preset condition to form an acetylacetamide-N-sulfonic acid triethylamine salt solution as an intermediate solution.

Optionally, the above method is carried out in a continuous reactor, and the continuous reactor is a fixed bed reactor, a continuous stirred tank reactor or a microchannel reactor.

The beneficial effects of the present application lie in that a solid superacid catalyst is used to replace the traditional organic acid catalyst, the solid superacid catalyst can not only promote the ammonium sulfamate solution and the diketene to carry out an acylation reaction smoothly, providing acylation reaction efficiency , but also quickly complete the hydrolysis reaction in a short time relying on the acidity provided by the solid superacid, without using acidic solution in the hydrolysis step, reducing the impurities remaining in the acesulfame, making the appearance of the final acesulfame product better, and significantly improving the use experience of the user; and on the other hand, the sulfur trioxide is dissolved in a solvent to form a cyclizing agent, so that the stability of the precursor cyclized product (ASH) of sulfur trioxide and acesulfame (ASK) is improved, the reaction moves forward, the conversion rate of the raw materials is improved, the yield of the cyclized product (ASH) is increased to a great extent, the yield of the final acesulfame product is further improved, and the production cost of acesulfame is reduced.

The foregoing description is merely an overview of the technical solutions of the present application. In order to make the technical means of the present application clearer to be understood so as to be implemented according to the contents of the description, and in order to make the above and other objects, features and advantages of the present application more comprehensible, specific embodiments of the present application are described below.

### Detailed Description of Embodiments

Exemplary embodiments of the present application will be described in more detail below. It should be understood that the present application may be implemented in various forms and should not be limited by the embodiments set forth herein. Rather, these embodiments are provided to enable a more thorough understanding of the present application and can fully convey the scope of the present application to those skilled in the art.

The conception of the present application is to provide a method for preparing acesulfame potassium which has a simple process and is suitable for continuous large-scale production, against the problem of bad appearance of the final product caused by the presence of acetic acid, and other problems of low yield and low product quality of the final acesulfame product due to easy decomposition of the precursor ASH of acesulfame ASK caused by instability of sulfur trioxide and the cyclized product ASH in the production process of acesulfame.

The method for preparing acesulfame potassium provided by the present application at least comprises step S110 to step A140:
An intermediate preparing step S110: adding triethylamine into a sulfamic acid solution, and carrying out an amination reaction to produce an ammonium sulfamate solution; and adding diketene to the obtained ammonium sulfamate solution, and carrying out an acylation reaction under the action of a solid superacid catalyst to obtain an intermediate solution.

The preparation of the intermediate is more meticulously divided into two small steps, first, preparing ammonium sulfamate, and then preparing the intermediate, i.e. acetylacetamide-N-sulfonic acid triethylamine salt, by reacting ammonium sulfamate with diketene.

The ammonium sulfamate is obtained by adding triethylamine to the sulfamic acid solution for amination reaction. Specifically, in some embodiments of the present application, the sulfamic acid is dissolved in a first solvent to prepare a first reaction solution; triethylamine is dissolved in a second solvent to prepare a second reaction solution, and the second reaction solution is added to the first reaction solution for amination reaction to form an ammonium sulfamate solution. The first solvent and the second solvent are inert organic solvents, such as dichloromethane, capable of providing a reaction environment for the amination reaction. Sulfamic acid and triethylamine carry out an exothermic reaction, in the reaction process, the heat generated will vaporize part of dichloromethane, the vaporized dichloromethane will leave the reaction system to take away the heat generated, and further, the vaporized dichloromethane can also be recycled.

When the first reaction solution and the second reaction solution are mixed, the second reaction solution is preferably gradually added dropwise into the first reaction solution, so that the reaction is more fully complete, not causing the concentration of part of the reactant to be too high and the reaction level to be too violent.

Specific embodiments for producing an ammonium sulfamate solution is given below, this embodiment is merely an exemplary description, and the specific production process of the ammonium sulfamate solution may be any one of the prior art. Sulfamic acid, the first dichloromethane, triethylamine and the second dichloromethane are weighed according to a preset dosage ratio, the first dichloromethane is added into a dried reaction kettle by opening the valve of the measuring tank for reactivity, and the stirring and circulating pump is started; and the sulfamic acid is fed from the feeding hole. The circulating valve is closed, the feeding valve is opened, the mixed material in the dissolving kettle is sent to a dry synthesis kettle, circulating water is used for cooling, and the first reaction liquid is obtained when the temperature of the reaction kettle is reduced to room temperature (about 20-25 °C).

In a process the same as the above, a second reaction solution of triethylamine dissolved in dichloromethane is obtained.

The second reaction solution was added dropwise to the first reaction solution, and at the end of the dropwise addition, the pH value is 7-9, it stands for 1 hour for reaction, and the material after the reaction is completed is an ammonium sulfamate solution.

After the sulfamic acid solution is obtained, the sulfamic acid solution is reacted with diketene to obtain an acetylacetamide-N-sulfonic acid triethylamine salt as an intermediate for preparing acesulfame.

In the prior art, the reaction between the sulfamic acid solution and diketene is carried out in the environment of acetic acid, and in the subsequent steps, the by-product caused by acetic acid is difficult to be completely removed from the final acesulfame product, and the by-product remaining in acesulfame caused by acetic acid not only makes the acesulfame not good in color, but also brings odour.

In the present application, solid superacid is used to replace the traditional acetic acid to effectively overcome this problem. The solid superacid can provide sufficient acidic sites for the acylation reaction, on one hand it can effectively catalyze ammonium sulfamate and diketene to carry out an acylation reaction smoothly, on the other hand the solid superacid will not be mixed into the reaction product, and a special treatment process is not needed subsequently, so that the post-treatment economy and time cost are saved; and the adverse effect on the final product appearance caused by the fact that acetic acid impurity not being removed remains in the final product in the prior art is avoided.

In order to increase the flash point of diketene, diketene is dissolved in a third solvent to prepare a third reaction solution, and the third solvent, such as dichloromethane, is an inert organic solvent capable of providing a reaction environment for an amination reaction. The reactor is filled with a solid superacid catalyst, the ammonium sulfamate solution and the third reaction solution are sequentially added to the reactor, and the reaction is carried out under preset conditions to form an acetylacetamide-N-sulfonic acid triethylamine salt solution as an intermediate solution.

In order to achieve the continuity of the reaction, in some embodiments of the present application, a continuous reactor may be selected to implement the present application, such as a fixed bed reactor, a continuous stirred tank reactor, or a microchannel reactor. A fixed bed reactor is taken as an example here to briefly describe the reaction process.

The fixed bed reactor is filled with a solid superacid as a catalyst, the fixed bed reactor is set to a preset working state, first the ammonium sulfamate solution is introduced into the fixed bed reactor, then the third reaction liquid is introduced into the fixed bed reactor in the same direction after the ammonium sulfamate solution flows normally, the contacting time of the two is within a preset condition by controlling the flow rate of the two, meanwhile the reaction temperature is also within a preset condition by controlling the heat exchange device of the fixed bed reactor, and the reaction can be ended when the preset reaction time is reached to obtain the acetylacetamide-N-sulfonic acid triethylamine salt solution product. Due to the characteristics of the fixed bed reactor, the present reaction can be continuously carried out, suitable for large-scale industrial production.

A sulfonation cyclization step S120: dissolving sulfur trioxide in a solvent to form a cyclizing agent solution; adding the cyclizing agent solution to the intermediate solution, and carrying out a sulfonation cyclization reaction to obtain a cyclized product solution.

Different from the direct use of sulfur trioxide in the prior art, in the present application sulfur trioxide is dissolved in a solvent to form a cyclizing agent solution, and the solvent may be, but is not limited to, an inert organic solvent such as dichloromethane. The solvent has a stabilizing effect on sulfur trioxide, so that sulfur trioxide is not easy to sublimate, and the cyclization reaction is carried out forward.

The cyclizing agent solution is added to the intermediate solution, and a sulfonation cyclization reaction is carried out to obtain a cyclized product solution. This reaction step is an exothermic reaction and preferably carried out at a lower temperature, and in order to enhance the controllability of the reaction and avoid part of the reaction being too violent, the cyclization agent solution can be gradually added dropwise into the intermediate solution with stirring to obtain a cyclized product solution, wherein the cyclized product is the precursor ASH of acesulfame.

A hydrolysis step S130: a hydrolysis agent is added to the cyclized product solution, and a hydrolysis reaction is carried out to obtain a hydrolysate solution.

Different from a conventional process in which an acidic solution is used for hydrolysis reaction, in the present application a hydrolysis agent is used to hydrolyze the cyclized product relying on the acidity of the solid superacid, which can significantly improve the rate of hydrolysis reaction, shorten the hydrolysis time, and reduce the amount of impurities that may be produced by hydrolysis along with the decrease of the hydrolysis time. In some embodiments of the present application, the hydrolysis agent is water or an ethanol aqueous solution, the hydrolysis performed by using the hydrolysis agent can significantly reduce the content of impurities in the cyclized product ASH, reduce the difficulty of subsequent acesulfame purification, and cut the cost of acesulfame purification.

A salt forming step S140: a potassium hydroxide solution is added to the organic phase of the hydrolysate solution to obtain acesulfame potassium.

This step is a conventional salt formation step, and specifically, a potassium hydroxide solution can be just added to the organic phase of the hydrolysate solution to obtain acesulfame potassium.

In conclusion, according to the present application, the solid superacid catalyst is adopted to replace the conventional organic acid catalyst combination technical solution, so that the appearance of the final acesulfame product is better, and the use experience of a user is remarkably improved. On the other hand, sulfur trioxide is dissolved in a solvent to form a cyclizing agent, the hydrolysis is carried out by using a hydrolytic agent in the hydrolysis step without the acidic condition in the prior art, significantly improving the stability of the precursor cyclized product (ASH) of acesulfame (ASK), making the reaction move forward, improving the conversion rate of the raw materials, greatly improving the yield of the cyclized product (ASH), further improving the yield of the final acesulfame product, and reducing the production cost of acesulfame.

### Types of the Solid Superacid Catalyst

The solid superacid refers to an acid that has an acidicity in excess of 100% sulfuric acid, for example, the acid strength is represented by Hammett acidity function H, the H0 value of 100% sulfuric acid is 11.93, and the acid of which H0<-11.93 is a superacid. The solid superacid is divided into two classes, one class contains halogen and fluorosulfonic acid resins as fluoride solid compounds, and the other class does not contain halogen, which is prepared by high-temperature combustion of sulfate radicals adsorbed on the surface of the metal oxide or hydroxide. In the present application, the type of the solid superacid is preferably the latter, i.e. SO₄²⁻/MₓO_{y} type superacid, wherein M represents a metal atom, and may specifically be Zn, Zr, Ti, Sn, etc. preferably SO₄²⁻/Fe₂O₃ type superacid catalyst.

In the present application, the amount of the solid superacid catalyst is not limited, and can be determined according to the specification of the reactor.

### Types and the Amount of the Hydrolytic Agent

In some embodiments of the present application, in the above method, the hydrolysis agent is deionized water or an ethanol aqueous solution, preferably an aqueous solution of ethanol, wherein the mass concentration of ethanol in the ethanol aqueous solution is preferably 30-65%. Through a large number of experiments, the inventors find that using a hydrolysis agent, especially an ethanol aqueous solution, and controlling the content of water in the hydrolysis agent, can obviously reduce the content of impurities in the acesulfame precursor ASH, reduce the difficulty of subsequent acesulfame purification, and cut the cost of acesulfame purification.

The amount of the hydrolysis agent is not limited in the present application, the amount of the hydrolysis agent may be determined according to the amount of sulfur trioxide, and specifically, in some embodiments of the present application, the molar ratio of the amount of sulfur trioxide to the content of water in the hydrolysis agent is 1: 1-4, and in other embodiments, 1: 1-1.5. That is, the amount of substance of water in the hydrolysis agent is preferably higher than the amount of the substance of sulfur trioxide.

### The amount of Sulfur Trioxide

The amount of sulfur trioxide is not limited in the present application, the amount of sulfur trioxide may be determined according to the amount of sulfamic acid. Specifically, considering economic factors, in some embodiments of the present application, the molar ratio of the amount of sulfamic acid to the amount of sulfur trioxide is 4-8: 1, and in some embodiments of the present application, the molar ratio of the amount of sulfamic acid to the amount of sulfur trioxide is 6: 1.

### Conditions for the Hydrolysis Reaction

In the present application, the conditions for the hydrolysis reaction are not limited, and any condition in which requirement for hydrolysis reaction can be met is just suitable. In some embodiments of the present application, in the hydrolysis reaction step, the reaction temperature is set to be 0 to 20° C, in some other embodiments, 10 to 20° C, and the reaction time is set to be 30-400 s. That is to say, the hydrolysis reaction step of the present application is preferably carried out at a lower temperature, and since dichloromethane can take away a large amount of the heat generated, therefor in the present application, temperature control is easier to be implemented, and any one of the prior art can just be used, such as an air condensation technology, a circulating water condensation technology and a heat exchange plate. Since the solid superacid catalyst is used in the present application, the reaction time of the hydrolysis reaction step can be significantly shortened, and the reaction can be completed more thoroughly for 30-400 s. If the reaction temperature is lower than 0°C and the reaction time is shorter than 30 s, the hydrolysis may not be completed, resulting in an incomplete hydrolysis level, no conversion of some cyclic compound, and a low degree of conversion of raw materials; if the reaction temperature is higher than 20°C and the reaction time is longer than 400 s, the reaction temperature is too high, the reaction time is too long, the time cost is increased, and the cyclized product is easy to decompose, which is not conducive to the development of the reaction towards the direction of hydrolysis reaction.

The amounts and the reaction conditions of other reaction materials not mentioned above can be referred to the prior art, for example, in Chinese patent document CN112142687 A, the molar ratio of sulfamic acid to diketene is n(sulfamic acid): n (diketene): n (triethylamine) = 1: 1.0-3.0: 1.0-4.0; the acylation reaction temperature is -40 to 15°C; the dropwise adding time is 10 min-300 min; and the reaction time is 1-15 h, and the like.

### Sources of Drugs or Reagents

In the present application, each drug or reagent may be self-made in a laboratory or factory, or a commercially available product may be used, which is not limited in the present application.

Preparation of an intermediate solution: an amination reaction step: 98 kg of sulfamic acid and a first dichloromethane are dissolved at a molar ratio of 1: 6, and the dissolving temperature is controlled to be about 20 to 25°C to obtain a dichloromethane solution of sulfamic acid, i.e. the first reaction solution. The dissolution may be in a continuous mixing device or in a reaction vessel.

Triethylamine and a second dichloromethane are dissolved at a molar ratio of 1: 1, and the dissolving temperature is controlled to be 10 to 30°C to obtain a second reaction solution, wherein the mass ratio of sulfamic acid to triethylamine is 1: 1.2. The second reaction solution is gradually added dropwise into the reaction kettle where the first reaction solution is located for mixing and stirring, the temperature of the system is controlled to be 20 to 30° C, the system is controlled to be weakly alkaline, and it is uniformly mixed to obtain an ammonium sulfamate solution.

An acylation reaction step: diketene and a third dichloromethane are dissolved in a molar ratio of 1: 1.5, and the dissolving temperature is controlled to be 10 to 20°C to obtain a third reaction solution.

After the solid superacid catalyst is fixed to the fixed bed reactor, the fixed bed reactor is started, and the circulating water is adjusted to make the circulating water work normally.

The ammonium sulfamate solution is introduced into the fixed bed reactor, after the ammonium sulfamate solution normally flows, the third reaction solution is introduced into the fixed bed reactor in the same direction as the ammonium sulfamate solution, and the amount of the ammonium sulfamate solution and the third reaction solution is controlled, so that the molar ratio of the sulfamic acid to the diketene is 1: 1.1. After the reaction starts, the temperature of the cooling water is adjusted as low as possible, and the temperature of the reaction system is controlled to be 20 to 35° C; and with the decline of the performance of the catalyst, the temperature can be slightly increased within the control range.

The intermediate solution of each of the embodiments and comparisons in the present application is prepared by the above methods if not specifically described, or following special descriptions if specifically described.

The reduced hydrolysis reaction time can improve the purity of the product, the final product acesulfame potassium is generally edible or medicinal, the impurities are generally calculated in ppm, and therefore the improvement of purity seems trivial but it is greatly beneficial to the improvement of the product quality. The hydrolysis reaction time of 30-400 seconds was observed to facilitate to improve the purity. The following embodiments give the reaction effect under different reaction conditions, especially within different hydrolysis times.

### Embodiment 1 (Embodiment 1A, Embodiment 1B, Embodiment 1C, Embodiment 1D, Embodiment 1E, Embodiment 1F, Embodiment 1G, Embodiment 1H)

Preparation of the intermediate solution: when the intermediate solution was prepared, superacid SO₄²⁻/Fe₂O₃ was used as a catalyst, and other steps referred to the preparation of the intermediate solution A.

A sulfonation cyclization step: sulfur trioxide was dissolved in dichloromethane to form a cyclizing agent solution, the cyclizing agent solution was added to the intermediate solution, and a sulfonation cyclization reaction was carried out to obtain a cyclized product solution.

A hydrolysis step: an ethanol aqueous solution as a hydrolysis agent was added to the cyclized product solution, and a hydrolysis reaction was carried out to obtain a hydrolysate solution.

A salt forming step: an ethanol solution of potassium hydroxide was added to the organic phase of the hydrolysate solution to obtain acesulfame potassium. Specific reaction conditions and reaction results refer to Table 1.

### Comparative Embodiment 1 (Comparative Embodiment 1A, Comparative Embodiment 1B, Comparative Embodiment 1C)

Preparation of the intermediate solution: when the intermediate solution was prepared, superacid SO₄²⁻/Fe₂O₃ was used as the catalyst, and other steps referred to the preparation of the intermediate solution A.

A sulfonation cyclization step: sulfur trioxide was dissolved in dichloromethane to form a cyclizing agent solution, the cyclizing agent solution was added to the intermediate solution, and a sulfonation cyclization reaction was carried out to obtain a cyclized product solution.

A hydrolysis step: an ethanol aqueous solution as a hydrolysis agent was added to the cyclized product solution, and a hydrolysis reaction was carried out to obtain a hydrolysate solution. The hydrolysis was carried out at a lower temperature in Comparative Embodiment 1 compared with that in Embodiment 1.

A salt forming step: an ethanol solution of potassium hydroxide was added to the organic phase of the hydrolysate solution to obtain acesulfame potassium. Specific reaction conditions and reaction results refer to Table 1.

Comparative Embodiment 2 (Comparative Embodiment 2A, Comparative Embodiment 2B, Comparative Embodiment 2C, Comparative Embodiment 2D, Comparative Embodiment 2E, Comparative Embodiment 2F, Comparative Embodiment 2G, Comparative Embodiment 2H)

Preparation of the intermediate solution: when the intermediate solution was prepared, no catalyst was used, and other steps referred to the preparation of the intermediate solution A.

A sulfonation cyclization step: sulfur trioxide was dissolved in dichloromethane to form a cyclizing agent solution, the cyclizing agent solution was added to the intermediate solution, and a sulfonation cyclization reaction was carried out to obtain a cyclized product solution.

A hydrolysis step: an ethanol aqueous solution as a hydrolysis agent was added to the cyclized product solution, and a hydrolysis reaction was carried out to obtain a hydrolysate solution.

A salt forming step: an ethanol solution of potassium hydroxide was added to the organic phase of the hydrolysate solution to obtain acesulfame potassium. Specific reaction conditions and reaction results refer to Table 1.

Comparative Embodiment 3 (Comparative Embodiment 3A, Comparative Embodiment 3B)

Preparation of the intermediate solution: when the intermediate solution was prepared, superacid SO₄²⁻/Fe₂O₃ was used as a catalyst, and other steps referred to the preparation of the intermediate solution A.

A sulfonation cyclization step: sulfur trioxide was dissolved in dichloromethane to form a cyclizing agent solution, the cyclizing agent solution was added to the intermediate solution, and a sulfonation cyclization reaction was carried out to obtain a cyclized product solution.

A hydrolysis step: an ethanol aqueous solution as a hydrolysis agent was added to the cyclized product solution, and a hydrolysis reaction was carried out to obtain a hydrolysate solution. The hydrolysis was carried out at a lower temperature in Comparative Embodiment 1 compared with that in Embodiment 1..

A salt forming step: an ethanol solution of potassium hydroxide was added to the organic phase of the hydrolysate solution to obtain acesulfame potassium. Specific reaction conditions and reaction results refer to Table 1.

Comparison 1 (Comparison 1A, Comparison 1B, Comparison 1C, Comparison 1D, Comparison 1E)

Preparation of the intermediate solution: when the intermediate solution was prepared, no catalyst was used, and other steps referred to the preparation of the intermediate solution A.

A sulfonation cyclization step: sulfur trioxide was dissolved in dichloromethane to form a cyclizing agent solution, the cyclizing agent solution was added to the intermediate solution, and a sulfonation cyclization reaction was carried out to obtain a cyclized product solution.

A hydrolysis step: water as a hydrolysis agent was added to the cyclized product solution, and a hydrolysis reaction was carried out to obtain a hydrolysate solution.

A salt forming step: an ethanol solution of potassium hydroxide was added to the organic phase of the hydrolysate solution to obtain acesulfame potassium. Specific reaction conditions and reaction results refer to Table 1.

**TABLE 1**

| | | Ratio of sulfur trioxide to water in the hydrolysis agent | Temperature of the hydrolysis reaction | Ethanol concentration in the ethanol aqueous solution | Time of the hydrolysis reaction (second) | Yield (%) |
|---|---|---|---|---|---|---|
| Embod iment 1 | Embodiment 1A | 1:1 | 0 | 65 | 60 | 87.9 |
| | Embodiment 1B | 1:1 | 10 | 65 | 40 | 93.2 |
| | Embodiment 1C | 1.5:1 | 20 | 30 | 30 | 94.7 |
| | Embodiment 1D | 1.5:1 | 20 | 65 | 30 | 95.4 |
| | Embodiment 1E | 1.5:1 | 20 | 10 | 30 | 88.5 |
| | Embodiment 1F | 2:1 | 20 | 30 | 30 | 91.4 |
| Compa rative Embod iment 1 | Comparative Embodiment 1A | 1:1 | -20 | 10 | 400 | 75.2 |
| | Comparative Embodiment 1B | 1:1 | -10 | 30 | 300 | 73.6 |
| | Comparative Embodiment 1C | 1.5:1 | -20 | 65 | 30 | 72.9 |
| | Comparative Embodiment 2A | 1:1 | -20 | 30 | 3000 | 73.6 |
| | Comparative Embodiment 2B | 1:1 | -10 | 65 | 3000 | 74.2 |
| | Comparative Embodiment 2C | 1:1 | 10 | 30 | 3000 | 76.3 |
| | Comparative Embodiment 2D | 1.5:1 | 20 | 65 | 3000 | 61.1 |
| | Comparative Embodiment 2E | 1.5:1 | -20 | 80 | 3000 | 71.9 |
| Compa rative Embod iment 2 | Comparative Embodiment 2F | 2:1 | -20 | 10 | 3000 | 63.2 |
| | Comparative Embodiment 2G | 3:1 | -20 | 80 | 3000 | 59.8 |
| | Comparative Embodiment 2H | 4:1 | -20 | 80 | 3000 | 55.6 |
| Compa rative Embod iment 3 | Comparative Embodiment 3A | 3:1 | 20 | 30 | 30 | 86.4 |
| | Comparative Embodiment 3B | 4:1 | 20 | 30 | 30 | 81.1 |
| Compa rison | Comparison 1A | 1:1 | -20 | / | 3000 | 52.2 |
| | Comparison 1B | 1:1 | -10 | / | 3000 | 53.4 |
| | Comparison 1C | 1:1 | 0 | / | 3000 | 58.1 |
| | Comparison 1D | 1.5:1 | 10 | / | 3000 | 62.2 |
| | Comparison 1E | 1.5:1 | 20 | / | 3000 | 57.6 |

| | | | | | | |
|---|---|---|---|---|---|---|
| Note: The method for calculating the yield of acesulfame potassium is based on diketene, i.e. the ratio of the actual production of acesulfame potassium to the theoretical production of potassium acesulfame based on diketene. | | | | | | |

It can be seen from Comparison 1 and Comparative embodiment 1 that in Comparative Embodiment 1 the temperature of the hydrolysis reaction is low compared with that of Comparison 1, the yield of acesulfame is also significantly lower than those of Embodiments, and therefore, the hydrolysis reaction temperature is 0°C to 20° C, which is a relatively ideal reaction temperature.

It can be seen from Comparison 1 and Comparative Embodiment 2 that no catalyst is used when preparing the intermediate solution in Comparison 1 and Comparative Embodiment 2, and it can be seen from Table 1 that the hydrolysis reaction requires a longer time (3000 s) to complete.

Comparative Embodiment 2 differs from Comparison 1 in that water is used as a hydrolysis agent in Comparison 1, while ethanol water is used as a hydrolysis agent in Comparative Embodiment 2, and it can be seen from Comparative Embodiment 2A and Comparison 1A, Comparative Embodiment 2B and Comparison 1B, and Comparative Embodiment 2D and Comparison 1E that the yields of acesulfame using an ethanol aqueous solution as a hydrolytic agent are all higher than those in embodiments using water as a hydrolytic agent in the case that other conditions are the same.

Compared with Comparative Embodiment 2, the solid superacid SO₄²⁻/Fe₂O₃ is used as a catalyst in Embodiment 1, and it can be seen from the reaction time, using solid superacid SO₄²⁻/Fe₂O₃ as a catalyst can significantly catalyze the subsequent hydrolysis reaction to carry out, improve the reaction efficiency, and shorten the hydrolysis reaction time.

It can be seen from Embodiment 1 that in the case that the solid superacid SO₄²⁻/Fe₂O₃ is used as a catalyst and meanwhile the ethanol aqueous solution is used as a hydrolysis agent, the reaction can be completed within an extremely short period of time, and the yield of acesulfame can reach 95.4%.

It can be seen from Embodiment 1 and Comparative Embodiment 3 that the difference between Comparative Embodiment 3 and Embodiment 1 is in that the ratio of sulfur trioxide to the water in the hydrolysis agent is different, and in the case that the ratio of sulfur trioxide to the water in the hydrolysis agent is 3: 1-4: 1, the yield of acesulfame potassium is decreased.

In the above embodiments, after each group of reaction is finished, the same salt forming process was adopted, the potassium hydroxide solution specifically the potassium hydroxide ethanol solution was added, salt formation and separation are performed to obtain an acesulfame crude product, and the purity of acesulfame is tested by using a high performance liquid chromatography method. After testing, the average purity of acesulfame of the first group of reactants is 93.3%, the average purity of acesulfame of the second group of reactants is 98.2%, and the average acesulfame purity of the third group of reactants is 98.8%. It can be seen from the results that a high-purity acesulfame crude product can be obtained by using a hydrolysis agent (water or an ethanol aqueous solution), and the purity of the acesulfame crude product can reach 90% or above; furthermore, using an ethanol aqueous solution and controlling the water content thereof can obviously reduce the amount of impurities in the acesulfame precursor ASH , i.e. reduce the content of impurities in the final acesulfame product, and reduce the difficulty and cost of subsequent acesulfame purification. It can be seen therefrom that using the ethanol aqueous solution as a hydrolysis agent can reduce the impurity content of acesulfame potassium, and obtain a high-quality acesulfame crude product.

To sum up, through repeated experiments of the inventors, the inventors find that in the case of using a solid superacid as a catalyst, the hydrolysis reaction time can be effectively reduced; as the hydrolysis time is reduced, the impurity content that may be generated by hydrolysis is reduced, and therefore a high-quality and efficient reaction can be maintained at a higher temperature (0-20° C) by using a solid superacid catalyst.

In continuous production, a slow reaction speed requires larger reaction equipment and more reactant inputs for the same yield, which means a higher risk for reactants not economical for production and in a large amount, and comprehensively considering yield, impurities and other cases, maintaining a longer time is not advantageous. Therefore, the solid superacid catalyst adopted in the present application to replace the conventional organic acid catalyst combination technical solution can shorten the hydrolysis reaction time, and make the appearance of the final acesulfame product better, in which under the condition of a primary refining, the color is not shown as conventional brownish yellow, but is shown as light yellow, so that the use feeling of a user is remarkably improved, and the value of the product is improved in the cases of medicinal use or high-grade food and the like; on the other hand, dissolving sulfur trioxide in a solvent to form a cyclizing agent, and using a hydrolysis agent to hydrolyze in the hydrolysis step without the acidic condition of the prior art, can significantly improve the stability of the precursor cyclized product (ASH) of acesulfame (ASK), make the reaction move forward, improve the conversion rate of the raw materials, improve the yield of the cyclized product (ASH) to a great extent, further improve the yield of the final acesulfame product, and cut the production cost of acesulfame.

The above are merely specific embodiments of the present application, and under the above teachings of the present application, a person skilled in the art may perform other improvements or modifications on the basis of the above embodiments. It should be understood by those skilled in the art that the above specific description is merely a better explanation of the purpose of the present application, and the scope of protection of the present application shall be subject to the scope of protection of the claims.

In addition, those skilled in the art will understand that although some embodiments described herein include certain features included in other embodiments rather than other features, combinations of features of different embodiments mean to be within the scope of the present application and form different embodiments. For example, in the following claims, any one of the claimed embodiments may be used in any combination.

## Claims

1. A method for preparing acesulfame potassium, wherein comprising:
an intermediate preparation step: adding triethylamine to a sulfamic acid solution, carrying out an amination reaction to produce an ammonium sulfamate solution, adding diketene to the obtained ammonium sulfamate solution, and carrying out an acylation reaction under the action of a solid superacid catalyst to obtain an intermediate solution;
a sulfonation cyclization step: dissolving sulfur trioxide in a solvent to form a cyclizing agent solution, adding the cyclizing agent solution to the intermediate solution, and carrying out a sulfonation cyclization reaction to obtain a cyclized product solution;
a hydrolysis step: adding a hydrolysis agent into the cyclized product solution, and carrying out a hydrolysis reaction to obtain a hydrolysate solution; and
a salt forming step: adding potassium hydroxide to the organic phase of the hydrolysate solution to obtain acesulfame potassium.

2. The method of claim 1, wherein the solid superacid catalyst is a SO₄²⁻/MₓO_{y} type catalyst, wherein M represents a metal atom.

3. The method of claim 2, wherein the solid superacid catalyst is a SO₄²⁻/Fe₂O₃ type catalyst.

4. The method of claim 1, wherein the hydrolysis agent is deionized water or an aqueous ethanol solution.

5. The method of claim 4, wherein the hydrolysis agent is the ethanol aqueous solution, wherein the mass concentration of ethanol in the ethanol aqueous solution is 30-65%.

6. The method of claim 1, wherein the reaction temperature of the hydrolysis reaction is 0 to 20° C, more preferably 10 to 20° C; and the reaction time of the hydrolysis reaction is 30-400 s.

7. The method of claim 1, wherein the molar ratio of the amount of the sulfamic acid to the amount of the sulfur trioxide is 4-8: 1, preferably 6: 1; and
the solvent is dichloromethane.

8. The method of claim 1, wherein the molar ratio of the amount of sulfur trioxide to the content of water in the hydrolysis agent is 1: 1-4, preferably 1: 1-1.5.

9. The method of claim 1, wherein the specific steps of the intermediate preparation step comprise:
dissolving sulfamic acid in a first solvent to prepare a first reaction solution;
dissolving triethylamine in a second solvent to prepare a second reaction solution, adding the second reaction solution into the first reaction solution, and carrying out an amination reaction to form an ammonium sulfamate solution;
dissolving diketene in a third solvent to prepare a third reaction solution; and
filling the reactor with a solid superacid catalyst, sequentially adding the ammonium sulfamate solution and the third reaction solution to the reactor, and reacting under a preset condition to form an acetylacetamide-N-sulfonic acid triethylamine salt solution as an intermediate solution.

10. The method of claim 1, wherein the method is carried out in a continuous reactor, and the continuous reactor is a fixed bed reactor, a continuous stirred tank reactor or a microchannel reactor.
